Europäisches Patentamt

European Patent Office

Office européen des brevets

(11) Publication number: **0 301 833 B1**

# EUROPEAN PATENT SPECIFICATION

(45) Date of publication of patent specification: **22.06.94**  (51) Int. Cl.[5]: **C12N 5/00**, C12M 1/00,
C12M 3/02

(21) Application number: **88306924.7**

(22) Date of filing: **27.07.88**

The file contains technical information submitted
after the application was filed and not included in
this specification

(54) **Culture method and apparatus therefor.**

(30) Priority: **29.07.87 JP 191359/87**

(43) Date of publication of application:
**01.02.89 Bulletin  89/05**

(45) Publication of the grant of the patent:
**22.06.94 Bulletin  94/25**

(84) Designated Contracting States:
**AT BE CH DE ES FR GB GR IT LI LU NL SE**

(56) References cited:
**EP-A- 0 071 999**
**DE-A- 3 247 610**
**FR-A- 2 559 500**

**PROCEEDINGS OF THE 4TH INTERNATIONAL
FERMENTATION SYMPOSIUM, T. GYOZO:
"Fermentation technologie today", Kyoto,
19-25 March 1972, proc. 5, Society of Fer-
mentation Technology, Osaka (JP); R.Y. RYU,
pp. 129-133&NUM;**

(73) Proprietor: **MITSUI PETROCHEMICAL INDUS-
TRIES, LTD.**
**2-5, Kasumigaseki 3-chome**
**Chiyoda-ku**
**Tokyo 100(JP)**

(72) Inventor: **Matsubara, Koichi**
**Mitsui Petrochemical Ind.Ltd.,**
**1-2 Waki 6-chome**
**Wakichou Kugagun, Yamaguchi(JP)**
Inventor: **Toshioka, Toshihiro**
**Mitsui Petrochemical Ind.Ltd.,**
**1-2 Waki 6-chome**
**Wakichou Kugagun, Yamaguchi(JP)**
Inventor: **Motoyama, Yoshio**
**Mitsui Petrochemical Ind.Ltd.,**
**1-2 Waki 6-chome**
**Wakichou Kugagun, Yamaguchi(JP)**

(74) Representative: **Myerscough, Philip Boyd et al**
**J.A.KEMP & CO.**
**14, South Square**
**Gray's Inn**
**London WC1R 5LX (GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

## Description

The invention relates to a method for producing berberine by culturing Ranunculaceae plant cells or tissue.

As conventional methods for tissue culturing plant cells, batch culture and continuous culture methods are known.

(1) Batch culture

The yield of the cultured tissue or cells in batch culture varies depending on the concentration of nutrients in the culture medium (ingredients in the culture medium). When the conventional batch culture media Linsmaier-Skoog, White and Nitch & Nitch are used, nutrients are consumed during culture. Thus, it is impossible to obtain the cultured cells or tissue at high concentration. As a method for increasing the concentration of the cultured cells or tissue, there has been proposed a method wherein the concentrations of nutrients are increased as compared with conventional methods. However, when the concentrations of nutrients are too high, there is a problem that the high concentrations interfere with the culture and, for example, the cultured material may be broken. Thus, the method for increasing the productivity of the cultured material by increasing the concentrations of nutrients has a limit, and the yield of the cultured material per unit capacity of the culture tank is low and the efficiency is poor.

(2) Continuous Culture

Conventional methods for continuous culture are described in, for example, "Fermentation Engineering (Hakko Kogaku)", vol. 61, pp117-128 (1983) (written in Japanese).

In continuous culture, the cells in the tank are kept under nearly the same conditions throughout the culture period. Therefore, culture can be carried out industrially, for example, by increasing the rate of growth or proliferation of the cells with a change in the culture conditions, or by increasing the content of the desired substance. Further, the culture period is long in continuous culture and cells can be cultured at a given high concentration, whereas in batch culture a cycle consisting of charging-sterilising-culturing-recovering-washing-charging must be repeated. Accordingly, the efficiency of continuous culture is high compared with that of batch culture.

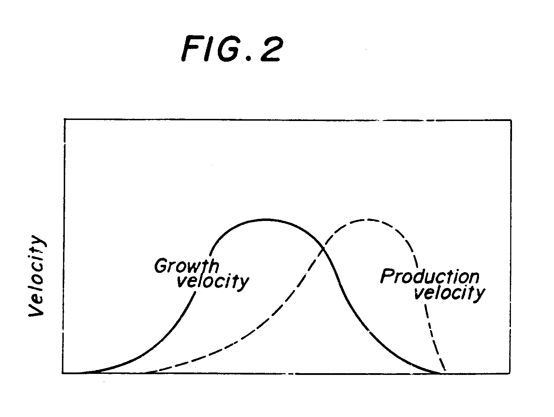

According to our studies, however, it has been found that there is a disadvantage in some cases that the productivity of continuous culture is lower than that of batch culture. We have studied the problem associated with conventional methods and found that the problem is caused by the fact that acceleration of cell growth and production of the desired substance occur at different times in almost all cases. We have found that when cells are cultured, the time required for reaching the peak rate of growth or proliferation of the cells differs from that required for reaching the peak rate of production of the desired substance, as shown in Fig. 2. The optimum conditions for growth of cells in liquid media are different from the optimum conditions for production of the useful substance. The invention seeks to eliminate the above-mentioned drawbacks.

The invention provides a method for continuously producing berberine by culturing Ranunculaceae plant cells or tissue, said method comprising:

(a) culturing said cells or tissue in liquid media in first and second culture tanks communicating with each other;

(b) maintaining the first culture tank under conditions suitable for the growth of said cells or tissue by maintaining in the growth medium a higher concentration of at least one of saccharide, phosphate and nitrogen source relative to the second culture tank;

(c) maintaining the second culture tank under conditions suitable for the production of berberine by maintaining in the production medium a lower concentration of at least one of saccharide, phosphate and nitrogen source relative to the first culture tank;

(d) supplying fresh growth medium to the first culture tank and simultaneously transferring part of the culture suspension containing said cells or tissue from the first culture tank to the second culture tank;

(e) adjusting the feed rate ($v$m1[l/day]) of the medium to the first culture tank, the specific growth velocity ($\mu$) of said cells or tissue in the first culture tank to 0.02 to 1.0 day$^{-1}$ and the amount (V) of the culture medium in the first culture tank such that $\mu < v$m1/V$ < 20\mu$; and

(f) removing part of the culture suspension containing said cells or tissue from the second culture tank and recovering berberine therefrom.

Preferably, culture medium suitable for the production of berberine is fed to the second culture tank, which medium differs from the culture medium fed to the first culture tank. Further, culture medium containing essentially no cells or tissue may be transferred from the first culture tank to the second culture tank, which medium is obtained by removing cells or tissue from culture suspension of the first culture tank. The cells or tissue may be removed by filtering culture suspension of the first culture tank.

According to the method of the invention, the culture medium in the first culture tank is kept under different conditions from those under which the culture medium in the second culture tank is kept. The culture medium in the first culture tank is conditioned so that it suits growth or proliferation of the cells or tissue, while the culture medium in the second culture tank is conditioned so that it suits production of berberine. Thus, the disadvantage of low productivity associated with conventional continuous culturing methods can be eliminated.

Thus, the invention allows productivity of berberine to be greatly improved by a continuous culture method composed of at least the following two stages:

a stage where cells or tissue placed under conditions for vigorous growth and other cells or tissue placed under conditions for rapid production are separately cultured in a continuous manner in a first culture tank and a second culture tank, respectively; and

a stage where an excess amount exceeding a predetermined amount of cells or tissue grown in the growth step conducted in the first culture tank is continuously or intermittently transferred to the production step conducted in the second culture tank, and culture suspension containing cells or tissue and berberine is discharged from the second culture tank and recovered.


BRIEF DESCRIPTION OF THE DRAWINGS

Figure 1 shows diagrammatically one embodiment of a culture apparatus which can be used in the invention.

Figure 2 is a graph illustrating the relationship between the rate of growth (proliferation) and the rate of production.

| 10: | first culture tank |
| 11, 21: | medium-feeding pipe |
| 20: | second culture tank |
| 15, 25: | discharge port |
| 30: | main communicating pipe |
| 32: | auxiliary communicating pipe |

In the present invention, at least two culture tanks are used.

The composition of the culture medium to be fed to the first culture tank is adjusted so as to accelerate the growth or proliferation of the tissue or cells to be cultured. The concentrations of ingredients such as sugar, phosphate and nitrogen source (which accelerate the growth of plant cells) are increased compared with those of other ingredients, and their feed rates are controlled so as to allow their concentrations in the culture solution to be kept within the optimum range. The preferred composition, the feed rate and the temperature of the culture medium, oxygen content and stirring conditions, etc. are individually determined depending on the type or the concentration of Ranunculaceae plant cells or tissue to be cultured as described hereinafter.

A culture medium suitable for increasing the concentration of berberine is fed to the second culture tank. Generally, a medium composition inhibiting the growth of cells gives favourable results. The culture medium composition which inhibits growth is a culture medium which contains main nutrient sources such as sugar, phosphate and nitrogen source at a relatively low concentration. However, the ingredients which play a role in decreasing or increasing the concentration of berberine vary depending on the type of Ranunculaceae plant, so that the concentrations of ingredients are determined according to the type of plant. The temperature of the culture medium, oxygen content, stirring conditions, etc. in the second culture tank are determined irrespective of those in the first culture tank.

These first and second culture tanks communicate with each other, for example, by a main communicating pipe. Part of the culture suspension stored in the first culture tank is continuously or intermittently transferred together with the cells or tissue to the second culture tank.

It is possible to feed fresh culture medium to the second culture tank. In some cases, feeding fresh culture medium to the second culture tank is preferred, depending on the type of plant to be cultured. In this case, it is believed that the second culture tank functions as an aging culture tank, because the culture suspension transferred from the first culture tank is used as such. When the value $\nu$m1/V (a value obtained by calculating the culture medium-feeding rate $\nu$m1 to the first culture tank in terms of the culture medium-renewing ratio) is larger than the specific growth velocity $\mu$, the culture solution containing the cells is transferred to the second tank at a velocity of $\mu$ and the culture solution from which the cells are removed is discharged at a velocity of $\nu$m1-$\mu$ from the tank to the outside. If desired, the discharged solution may be fed to the second tank to renew the culture solution in the second culture tank.

The definitions of the culture medium-renewing ratio and the specific growth (proliferation) velocity will be described in more detail hereinafter.

If desired, one or more intermediate culture tanks may be provided on the way of the main communicating pipe and the intermediate tanks may be conditioned between the conditions of the first and second culture tanks.

Examples of the Ranunculaceae plants which can be cultured include plants such as Coptis japonica and Larix leptolepis.

The cells or tissue of these plants are cultured in liquid media. The liquid media contain an inorganic substance, a carbon source and a plant hormone as essential ingredients. In addition thereto, the liquid media contain vitamins and optionally amino acids. Examples of the inorganic substance include nitrogen, phosphorus, potassium, sodium, calcium, magnesium, sulfur, iron, manganese, zinc, boron, molybdenum, chlorine, iodine and cobalt in the form of inorganic salts of these elements. Typical examples of the inorganic salts containing said elements include potassium nitrate, sodium nitrate, ammonium nitrate, ammonium chloride, potassium chloride, calcium chloride, dipotassium hydrogenphosphate, sodium dihydrogenphosphate, magnesium sulfate, magnesium chloride, sodium sulfate, ferrous sulfate, ferric sulfate, manganese sulfate, copper sulfate, sodium molybdate, molybdenum oxide, potassium iodide, zinc sulfate, boric acid and cobalt chloride.

Examples of the carbon source include carbohydrates such as sucrose, their derivatives, organic acids such as fatty acids, and primary alcohols such as ethanol.

Examples of the plant hormones or phytohormones include auxins such as indoleacetic acid (IAA), naphthaleneacetic acid (NAA), p-chlorophenoxyisobutyric acid and 2,4-dichlorophenoxyacetic acid (2,4-D); and cytokinins such as kinetin, zeatin and benzyladenine.

Examples of the vitamins which can be used in the liquid medium include biotin, thiamine (vitamin $B_1$), pyridoxine (vitamin $B_6$), pyridoxal, pyridoxamine, calcium pantothenate, ascorbic acid (vitamin C), inositol, nicotinic acid, nicotin amid and riboflavin (vitamin $B_2$).

Examples of the amino acids which can be used in the liquid medium include glycine, alanine, glutamic acid, cysteine, tyrosine and lysine.

It is preferred that the liquid medium of the present invention contains from about 0.1 $\mu$M to about 100 mM of the inorganic substance, from about 1 g to about 100 g of the carbon source per liter, from 0.01 $\mu$M to 1000 $\mu$M, preferably 1 $\mu$M to 100 $\mu$M of the plant hormone, from about 0.1 mg to about 150 mg of the vitamin per liter and from 0 to about 1000mg of the amino acid per liter.

Examples of the liquid medium include media prepared by adding the carbon source, the plant hormone and optionally, said vitamins and said amino acids to conventional liquid medium such as Murashige & Skoog ('62) medium, Linsmaier-Skoog (RM-1965) medium, White ('63) medium, Gamborg's B-5 medium and Mitsui M-9 medium which have been conventionally used for the tissue culture of plants.

The compositions of conventional culture media described above are described in "New Plant Tissue Culture (Shin Shokubutsu Soshiki Baiyo)", PP.386-391 (1979) by Takeuchi, Nakajima and Furuya, published by Asakura Shoten (written in Japanese).

It is preferred that culture is carrired out under the following conditions using the above-described liquid media.

(1) In the method for tissue culture of the present invention, the culture is carried out under such conditions that the concentration of the cultured cells or tissue based on the amount of the liquid medium is 10 to 500 [g/ℓ], preferably 20 to 400 [g/ℓ] during the period of the tissue culture. Since the cells or tissue is grown or proliferated and the amount thereof is increased during the period of the culture, an appropriate amount of the cells or tissue is optionally taken out from the culture tank during culture so as to allow the concentration of the cells or tissue in the culture tank to be kept in the range described above. The weight of the cultured cells or tissue to be taken out is fresh weight measured in a wetted state. The fresh weight is the weight of the cultured tissue measured by the following manner. Namely, the cultured tissue is fed into Nutsche funnel having a filter cloth put thereon, filtration is carried out for five minutes by means of a water-jet pump and the weight of the cultured cells or tissue, from which the culture solution is removed, is measured. The so-obtained fresh weight varies depending on the type of plant, but it generally has a water content of 80 to 95 wt.%.

The term "cultured cells or tissue" as used herein means substances obtained by culturing cells or tissue pieces of plants. Examples of the tissue pieces include stem top, stems, leaves, flowers, seeds and roots as well as callus derived from the tissue pieces and cultured cells. The cultured tissue can be used as a raw material for tissue culture. When the concentration of said cultured cells or tissue during culture is lower than 10 g/l the yield of cultured cells or tissue per unit capacity of the culture tank is low. Thus, the efficiency of the culture is low as in conventional methods and such a low concentration is not preferred. When the concentration of the cultured tissue is higher than 500 g/l, the concentration of the slurry is increased, stirring and mixing of the culture solution are difficult and oxygen cannot be supplied efficiently. As a result, death of the cultured tissue occurs, and normal growth and proliferation of the cultured cells or tissue are liable to become difficult. When stirring is vigorously carried out to supply a sufficient amount of oxygen, the cultured cells or tissue undergo breakage and damage and continuing culture becomes difficult. Thus, a concentration of higher than 500 g/l is not preferred. Accordingly, culture is carried out at a concentration of cultured cells or tissue from 10 to 500 g/l.

As described above, culture is carried out while the concentration of the cultured cells or tissue is kept in the range defined above. To meet the above requirement an appropriate amount of the cultured cells or tissue is optionally drawn out so that the concentration of the cultured tissue in the first and second culture tanks does not exceed the range defined above. Even when the concentration of the cutured cells or tissue is increased during the period of culture by growth thereof, it is not necessary to draw out part of the cultured tissue from the culture tank, unless the concentration exceeds the range described above.

It is not always necessary to keep the concentration of the cultured cells or tissue in the first and second culture tanks in the range of 10 to 500 g/l from the beginning of culture. For example, the concentration of the tissue pieces or the cultured cells to be charged into both tanks may be less than 10 g/l based on the amount of liquid medium. Culture may be started and the cells or tissue allowed to proliferate during culture, so that the concentration of cultured cells or tissue increases to 10 to 500 g/l.

(2) Each liquid medium containing nutrients at controlled concentrations is continously or intermittently fed to each culture tank, while the culture suspension is continuously or intermittently drawn out from the other side of each culture tank so as to meet the requirements of (1) above, that is, to allow the concentration of the cultured cells or tissue to be kept in the range of 10 to 500 g/ℓ. If desired, the cells or tissue are filtered off or precipitated, and culture is conducted while renewing the culture medium in a culture tank by drawing out the resulting culture solution containing no cells or tissue. The renewal of the culture medium may be conducted continuously or intermittently.

The nutrients described above are ingredients used in the liquid medium of the present invention and include inorganic substances, carbon sources, plants hormones, vitamins and amino acids.

The term "liquid medium containing nutrients at controlled concentrations" as used herein means a liquid medium containing the inorganic substancs in an amount of from about 0.1 $\mu$M to about 100 mM, the carbon source in an amount of from about 1 to about 100 g/ℓ, the plant hormones in an amount of from about 0.01 to 1000 $\mu$M, preferably 1 to 100 $\mu$M, the vitamins in an amount of about from 0.1 to about 150 mg/ℓ and the amino acids in an amount of from 0 to about 1000 mg/ℓ. As the liquid medium to be fed to the culture tanks, it is preferred to use a fresh liquid medium. However, the liquid medium removed from each culture tank may be recycled after adjusting the concentration of the nutrients. When the liquid medium is to be recycled, it is desirable that waste matters formed by the metabolism of the cells in the culture solution are removed.

Culture is carried out under the condition that the relationship between the medium-renewing ratio (defined by $\nu$m1/V [day$^{-1}$] or $\nu$t1/V [day$^{-1}$] and the specific growth velocity $\mu$ [day$^{-1}$] is kept in the range of $\mu \leq \nu$m1/V or $\nu$t1/V < 20 $\mu$ wherein V represents the amount in liters (ℓ) of the culture solution in each culture tank, $\nu$m1 represents the amount of the liquid medium to be fed to the first culture tank and $\nu$t1 represents the amount in (ℓ/day) of the liquid medium to be fed to the second culture tank. The specific growth velocity $\mu$ [day$^{-1}$] is an amount defined by the following method. Both $\nu$m1 and $\nu$t1 are hereinafter referred to as $\nu$ as a whole.

When the cultured cells or tissue is not discharged from the tank to the outside, the specific growth velocity $\mu$ is defined by the following formula:

$$\mu = \frac{\ell n \, \dfrac{Xt}{Xo}}{t}$$

wherein Xo represents the amount of the cultured cells or tissue at the time to of culture, and Xt represents the amount of the cultured cells or tissue after lapse of time t hours (day).

When the proliferated cultured cells or tissue is discharged from the tank to the outside, while keeping the amount Xo of the cultured cells or tissue, the specific growth velocity $\mu$ is defined by the following formula:

$$\mu = \frac{Yt}{Xo \cdot T}$$

wherein Yt represents the amount of the cultured cells or tissue discharged from the tank after the time t hours (day).

The velocity $\mu$ has a dimension of $[\text{time}^{-1}]$ in terms of physics and a larger $\mu$ value means that the velocity of the growth of the cultured cells or tissue is higher.

The value of the specific growth velocity $\mu$ varies depending on the type of the cultured cells or tissue of the plant, but is usually in the range of 0.02 to 1.0 $[\text{day}^{-1}]$.

When the medium-renewing ratio ($v/V$) is smaller than the specific growth velocity $\mu$ (the case where $v/V < \mu$), the concentration of the cultured tissue is gradually increased and it becomes difficult to keep the concentration constant.

When the medium-renewing ratio ($v/V$) is much larger than the specific growth velocity $\mu$ (the case where $20\mu \leqq v/V$), the cultured cells or tissue has an adverse effect, for example, blackening and death thereof are liable to occur. Thus, culture is conducted by adjusting the medium-renewing ratio ($v/V$) to a value within the range of $\mu \leqq v/V < 20\mu$.

As mentioned above, the first culture tank is conditioned so as to be suited to the growth of the plant material to be cultured. Generally, the temperature, the pH, the concentration of dissolved oxygen, the composition of the liquid medium, the medium-renewing ratio, the cell density, etc. are determined according to the type of the plant.

When cells or tissue of Coptis japonica is used, the optimum conditions are such that the temperature is 23 to 26°C, the pH is 4 to 6, the concentration of dissolved oxygen is 4 to 10 ppm and the culture media compositions A, C and D shown in Table 1 are used. Further, cell density is 50 to 500 g/l, preferably 100 to 400 g/l and the medium-renewing ration is 5 to 50% by volume per day.

As mentioned above, the second culture tank is conditioned so as to be suited to the production of berberine.

In the case that the cells or tissue of Coptis japonica is used to produce berberine, the concentration of dissolved oxygen is 4 to 15 ppm and culture medium having the composition I given in Table 1 is used.

Table 1

| Component | (Unit) | I | D | C | A |
|---|---|---|---|---|---|
| $NH_4NO_3$ | mM | 12 | 12 | 21 | 21 |
| $KNO_3$ | mM | 10 | 10 | 19 | 19 |
| $NaNO_3$ | mM | - | - | - | - |
| $CaCl_2 \cdot 2H_2O$ | mM | 2.9 | 2.9 | 3 | 3 |
| $MgSO_4 \cdot 7H_2O$ | mM | 2.5 | 2.5 | 3 | 3 |
| $KH_2PO_4$ | mM | - | 2.5 | 1.3 | 1.3 |
| $KI$ | μM | 5 | 5 | 5 | 5 |
| $H3BO_3$ | μM | 100 | 100 | 100 | 100 |
| $MnSO_4 \cdot 4H_2O$ | μM | 100 | 100 | 100 | 100 |
| $ZnSO_4 \cdot 7H_2O$ | μM | 30 | 30 | 30 | 30 |
| $Na2MoO_4 \cdot 2H_2O$ | μM | 1 | 1 | 1 | 1 |
| $CuSO_4 \cdot 5H_2O$ | μM | 1 | 1 | 1 | 0.1 |
| $CoCl_2 \cdot 6H_2O$ | μM | 0.1 | 0.1 | 0.1 | 0.1 |
| $Na_3EDTA$ | μM | 90 | 90 | 100 | 100 |
| $FeSO_4 \cdot 7H_2O$ | μM | 90 | 90 | 100 | 100 |
| $KCl$ | mM | - | - | - | - |
| Inositol | ppm | 100 | 100 | 100 | 100 |
| Thiamine | ppm | 0.4 | 0.4 | 0.4 | 0.4 |
| Glycine | ppm | - | - | - | - |
| L-glutamine | ppm | - | - | - | - |
| Nicotinic acid | ppm | - | - | - | - |
| Pyridoxine | ppm | - | - | - | - |
| IAA(indoleacetic acid) | μM | - | - | 1 | 1 |
| Kinetin | μM | - | - | 10 | 10 |
| NAA(naphthaleneacetic acid) | μM | - | 10 | - | - |
| BA(benzyladenine) | μM | - | 0.01 | - | - |
| 2,4-D(2,4-dichlorophenoxyacetic acid) | μM | - | - | - | - |
| Sucrose | % | 1 | 2 | 3 | 3 |

It is preferred that the culturing method of the invention is carried out using a culture apparatus described below.

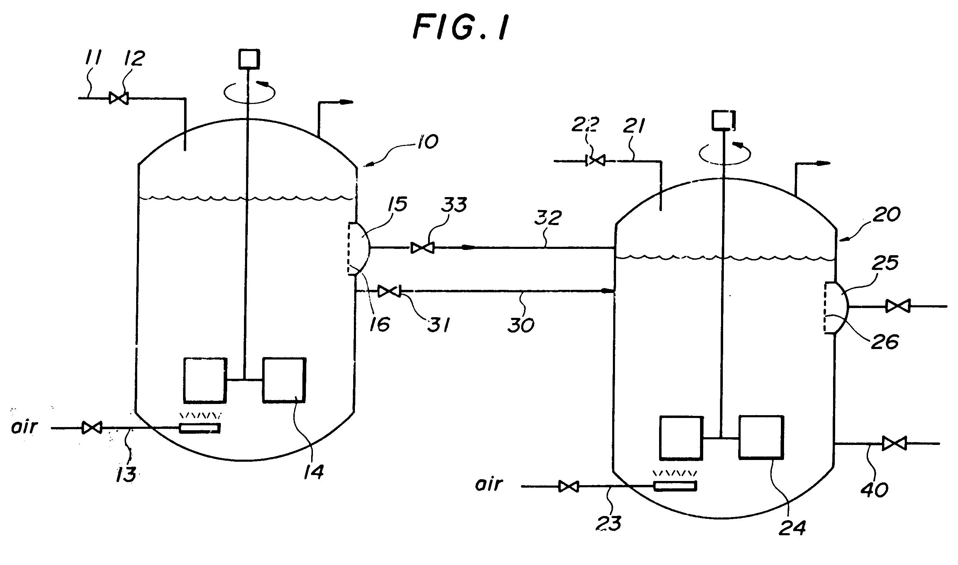

Referring to Fig. 1, the culture apparatus is provided with at least two culture tanks composed of a first culture tank 10 and a second culture tank 20. The culture tanks 10 and 20 communicate with each other continuously or intermittently through a main communicating pipe 30. Culture medium in the first culture tank 10 is transferred together with cells or tissue from the first culture tank to the second culture tank through said pipe 30. In one embodiment, the transfer of the culture suspension is made by means of a valve provided on the way of the main communicating pipe 30.

A liquid medium is stored in each of the first and second culture tanks. The conditions for each culture medium are controlled by each of a first and second culture medium condition-setting means so as to be suited to the growth of the cells or tissue and to the production of berberine.

In the embodiment shown in Fig. 1, as the culture medium condition-setting means there are used culture medium supply pipes 11 and 21 through which the culture media containing nutrients as described above are continuously or intermittently fed to the first and second culture tanks 10 and 20, respectively. The amount of culture medium fed through each of pipes 11 and 21 is controlled by means of each of valves 12 and 22 provided on the pipes 11 and 21, respectively.

The culture medium condition-setting means include a means for adjusting the composition of the culture medium solution; a means for controlling the temperature of the culture medium (not shown); air supply pipes 13 and 23 for adjusting the content of oxygen to be contained in the culture medium; stirring means 14 and 24 for stirring the culture solution, etc. The culture medium conditions are set differently between the first culture tank 10 and the second culture tank 20 by the culture condition-setting means. The culture medium in the first culture tank 10 is conditioned so as to be suited to the growth or proliferation of the cells or tissue, while the culture medium in the second culture tank 20 is conditioned so as to be suited to the production of berberine.

The culture tanks 10 and 20 are provided with discharge ports 15 and 25, respectively. Filters 16 and 26 are fixed to the discharge ports 15 and 25, respectively. Culture media containing no cultured cells or tissue from cultured mixtures can be discharged through the discharge ports from the tanks to the outside. As the filters 16 and 26, a net made of a metal, a synthetic resin, or a cloth having pores, made of a natural or synthetic fiber can be used. The mesh size or the pore size is such that the cultured cells or tissue is not passed therethrough, but the culture media alone can be passed therethrough. In case that a filter composed of a net is used, two or more sheets of filters may be superposed with each other, if desired. The discharge ports 15 and 25 having the filters fixed thereto may be provided at any position of the culture tanks 10 and 20, provided that the openings thereof are brought into contact with the culture suspensions. However, since renewal of the culture solution should be efficiently carried out, it is desirable that each of the discharge ports 15 and 25 is positioned at a point which is far away from each of the inlet ports (open ends of pipes 11 and 21) for introducing culture medium. If desired, an insert tube may be used as a cell setting tube in place of the filter, said tube having such a diameter that it gives a flow rate of the solution, that is, a discharge velocity lower than the velocity at which the cells or tissue settle out.

In the embodiment shown in Fig. 1, the discharge port 15 provided on the first culture tank 10 is allowed to communicate with the second culture tank 20 through an auxiliary communicating pipe 32. The communication is controlled by means of a valve 33 provided on the pipe 32.

The second culture tank 20 of the culture apparatus has a discharge tube 40 for drawing out the culture suspension (culture medium and cultured cells or tissue) after completion of culture.

As the aforementioned stirring means, there may be used a gyroscopic rotating culture tank which scarcely damages the cells or tissue by the action of an external force such as shearing force, described in our-copending Japanese Patent Application No. 103938/1984, or a rotating cylinder type culture apparatus described in Japanese Patent Laid-Open Publn. No. 134989/1983.

One or more intermediate culture tanks may be provided on the way of the main communicating pipe 30 and the culture medium stored in the intermediate tank is conditioned so that the culture condition thereof is set between those of the first and second culture tanks 10 and 20.

According to the culture method of the invention, the yield of cultured cells or tissue per unit capacity of the tank can be increased as compared with conventional methods. Thus, when culture is carried out on an industrial scale, the cultured cells or tissue can be produced in an economically advantageous manner at a high efficiency as compared with conventional methods.

The following Examples will further illustrate the present invention in more detail.

Example 1

There were used a first culture tank which had an empty volume of 4.5 $m^3$ and was provided with a 100-mesh stainless steel filter having a filtering area of 1 $m^2$ and a sparger having a pore size of 2 $\mu$ and a surface area of 0.5 $m^2$, and a second culture tank which had an empty volume of 5.5 $m^3$ and was provided with a filter. An apparatus having these two tanks was sterilized with steam at 125°C for 30 minutes. 3$m^3$ of a Linsmaier-Skoog liquid medium (hereinafter referred to as LS medium) containing 3 % sucrose, which was sterilized with a heat exchanger type continuous sterilizer, was placed in the first tank which was then kept at 25°C.

The cultured cell of Coptis japonica (obtained by culturing in the LS medium for 14 days) as seed cell was fed to the first tank in such an amount as to give a concentration of 4 g/ℓ. While introducing air of 0.05vvm thereinto, the culture was carried out for 10 days to obtain a culture solution containing cell at a concentration of 15 g/ℓ. Prom the seventh day, the culture medium D shown in Table 1 was fed to the first tank at intervals of two days in such an amount that the amount of sucrose in the culture solution was kept at a concentration of 0.5 to 1 %. The culture solution was passed through the filter to obtain an equal amount of the culture solution containing no cell. The solution was transferred to the second tank and the culture solution in the first tank was kept so as to amount to 3 $m^3$. From the twelfth day, the culture mixture (a culture solution containing cells a substance to be cultured) in an amount corresponding to proliferated cell was transferred to the second tank at intervals of about one hour so as to keep the cell in the culture solution in the first tank at a concentration of 14 to 16 g/ℓ. In addition to this operation, an excess amount of the culture solution (corresponding to an amount exceeding 3 $m^2$) in the first tank was transferred through the filter to the second tank. In the second tank, the cell-free culture solution obtained by passing it through the filter was discharge from the tank to keep the amount of the culture solution at a level of 4 $m^3$. In this

8

way, the concentration of the cell reached 15 g/ℓ on the nineteenth day.

The transferring of the cell-free culture solution from the first tank to the second tank was then stopped and the drawing-out of the culture solution containing the cell was started to keep the cell in the second tank at a concentration of from 13 to 17 g/ℓ. In this state, the culture was continued for about two months. During the period of this culture, a small amount of the cultured mixture was sampled from each of the first and second tanks. The samples were analyzed to determine the growth rate of the cell and the content of berberine, and productivity was calculated from the mean value thereof. In this example the productivity was calculated on the basis of the culture solution of 7 m³ which is the sum of the amounts in the first and second tanks.

The results are shown in Table 2.

Example 2

The procedure of Example 1 was repeated except that the concentration of the cell was set to 30 g/ℓ and the feeding of the cell-free culture solution drawn out from the first tank to the second tank was continued until the culture was terminated. On the eighteenth day after seed cell charging, the cell concentration was from 28 to 32 g/ℓ. From the 26th day, the drawing-out of the cell from the second tank was started.

The results are shown in Table 2.

EXample 3

The procedure of Example 1 was repeated except that the concentration of the cell was set to 70 g/ℓ and a LS medium containing 1 % sucrose and neither phosphoric acid nor plant hormone was fed to the second tank so as to give the culture solution containing the sucrose at a concentration of from 0 to 0.2 % in the second tank.

On the 26th day after seed cell charging, the cell concentration reached 66 to 73 g/ℓ, and the drawing-out of the cell from the second tank was started from 34th day.

The results are shown in Table 2.

Comparative Example 1

The first culture tank used in Example 1 was used and the culture was started in a similar manner to that described in Example 1. On the twelfth day, the cell concentration reached 15 g/ℓ. The cultured mixture in an amount corresponding to grown cell was then drawn out at intervals of about one hour and recovered so as to keep the cell at a concentration of from 14 to 16 g/ℓ. The cell/free culture solution was discharged through the filter while controlling the discharge rate to keep the amount of the culture solution at a level of 3 m³.

The results are shown in Table 2.

Comparative Examples 2 and 3

The procedure of Comparative Example 1 was repeated except that the cell concentrations were 30 g/ℓ and 70 g/ℓ, respectively.

The results are shown in Table 2.

EP 0 301 833 B1

Table 2

| | Example 1 | Example 2 | Example 3 |
|---|---|---|---|
| First tank | | | |
| Cell concn. (kg/m$^3$) | 15 | 30 | 70 |
| Specific growth velocity $\mu$ (1/day) | 0.23 | 0.24 | 0.24 |
| Second tank | | | |
| Cell concn. (kg/m$^3$) | 15 | 30 | 70 |
| Specific growth velocity $\mu$ (1/day) | 0.05 | 0.05 | 0.07 |
| Ratio of berberine(%) | 10 | 9 | 8 |
| Yield of cell (Kg/day) | 13.4 | 27.6 | 70 |
| Yield of berberine(Kg/day) | 7.3 | 2.5 | 5.6 |
| Yield of berberine per unit volume (Kg/m$^3 \bullet$ day) | 0.19 | 0.36 | 0.8 |
| | Comp. Example 1 | Comp. Example 2 | Comp Example 3 |
| Cell concn. (kg/m$^3$) | 15 | 30 | 70 |
| Specific growth velocity $\mu$ (1/day) | 0.18 | 0.21 | 0.17 |
| Ratio of berberine(%) | 4.6 | 3.9 | 4.8 |
| Yield of cell (Kg/day) | 8.1 | 18.9 | 35.7 |
| Yield of berberine(Kg/day) | 0.37 | 0.74 | 1.71 |
| Yield of berberine per unit volume (Kg/m$^3 \bullet$ day) | 0.12 | 0.25 | 0.57 |

**Claims**

**1.** A method for continuously producing berberine by culturing Ranunculaceae plant cells or tissue, said method comprising:

(a) culturing said cells or tissue in liquid media in first and second culture tanks communicating with each other;

(b) maintaining the first culture tank under conditions suitable for the growth of said cells or tissue by maintaining in the growth medium a higher concentration of at least one of saccharide, phosphate and nitrogen source relative to the second culture tank;

(c) maintaining the second culture tank under conditions suitable for the production of berberine by maintaining in the production medium a lower concentration of at least one of saccharide, phosphate and nitrogen source relative to the first culture tank;

(d) supplying fresh growth medium to the first culture tank and simultaneously transferring part of the culture suspension containing said cells or tissue from the first culture tank to the second culture tank;

(e) adjusting the feed rate (vm1[l/day]) of the medium to the first culture tank, the specific growth velocity ($\mu$) of said cells or tissue in the first culture tank to 0.02 to 1.0 day$^{-1}$ and the amount (V) of the culture medium in the first culture tank such that $\mu < vm1/V < 20\mu$; and

(f) removing part of the culture suspension containing said cells or tissue from the second culture tank and recovering berberine therefrom.

**2.** A method according to claim 1, wherein there is fed to said second culture tank the culture medium suitable for the production of berberine, which medium differs from the culture medium fed to the first culture tank.

**3.** A method according to claim 1 or 2 further comprising transferring culture medium containing essentially no cells or tissue from the first culture tank to the second culture tank, which medium is

10

obtained by removing cells or tissue from culture suspension of the first culture tank.

4. A method according to claim 3, wherein the cells or tissue are/is removed by filtering culture suspension of the first culture tank.

**Patentansprüche**

1. Verfahren zur kontinuierlichen Herstellung von Berberin durch Kultivierung von Ranunculaceae-Pflanzenzellen oder -gewebe, wobei das Verfahren folgendes umfaßt:

(a) Kultivierung der Zellen oder des Gewebes in flüssigem Medium in einem ersten und einem zweiten Kulturtank, die miteinander in Verbindung stehen;

(b) Halten des ersten Kulturtanks unter Bedingungen, die zum Wachstum der Zellen oder des Gewebes geeignet sind, indem im Vergleich zum zweiten Kulturtank im Wachstumsmedium eine höhere Konzentration mindestens einer Saccharid-, Phosphat- und Stickstoffquelle aufrechterhalten wird;

(c) Halten des zweiten Kulturtanks unter Bedingungen, die zur Herstellung von Berberin geeignet sind, indem im Vergleich zum zweiten Kulturtank im Herstellungssmedium eine geringere Konzentration mindestens einer Saccharid-, Phosphat- und Stickstoffquelle aufrechterhalten wird;

(d) Zuführen von frischem Wachstumsmedium zum ersten Kulturtank und gleichzeitiges Überführen eines Teils der die Zellen und das Gewebe enthaltenden Kultursuspension aus dem ersten Kulturtank zum zweiten Kulturtank;

(e) Einstellen der Eintragrate (vml [l/Tag]) des Mediums in den ersten Kulturtank, der spezifischen Wachstumsgeschwindigkeit ($\mu$) der Zellen oder des Gewebes im ersten Kulturtank auf 0,02 bis 1,0 Tag$^{-1}$ und der Menge (V) des Kulturmediums im ersten Kulturtank, so daß $\mu <$ vml/V $< 20\ \mu$ gilt;

(f) Entfernen eines Teils der die Zellen und das Gewebe enthaltenden Kultursuspension aus dem zweiten Kulturtank und Gewinnen des Berberins daraus.

2. Verfahren gemäß Anspruch 1, wobei in den zweiten Kulturtank das zur Herstellung von Berberin geeignete Kulturmedium eingetragen wird, welches Medium sich von dem Kulturmedium unterscheidet, das in den ersten Kulturtank eingetragen wird.

3. Verfahren gemäß Anspruch 1 oder 2, das ferner das Überführen von Kulturmedium, das im wesentlichen keine Zellen oder Gewebe enthält, von dem ersten Kulturtank zum zweiten Kulturtank umfaßt, welches Medium durch Entfernen der Zellen oder des Gewebes aus der Kultursuspension des ersten Kulturtanks erhalten wird.

4. Verfahren gemäß Anspruch 3, worin die Zellen oder das Gewebe durch Filtrierung der Kultursuspension des ersten Kulturtanks entfernt werden/wird.

**Revendications**

1. Procédé de production en continu de berbérine par culture de cellules ou de tissus de plantes appartenant à la famille de renonculacées, procédé comprenant :

(a) le fait de cultiver lesdits tissus ou cellules, dans un milieu liquide, dans une première et dans une seconde cuves de culture communiquant entre elles ;

(b) le fait de maintenir la première cuve de culture dans des conditions appropriées pour la croissance desdits tissus ou cellules, en maintenant le milieu de culture à une concentration plus élevée d'au moins une source de saccharide, de phosphate et d'azote, par rapport à la seconde cuve de culture ;

(c) le fait de maintenir la seconde cuve de culture dans des conditions appropriées pour la production de la berbérine, en maintenant le milieu de production à une concentration inférieure d'au moins une source de saccharide, de phosphate et d'azote, par rapport à la première cuve de culture ;

(d) la fait d'apporter du milieu de culture neuf à la première cuve de culture et de transférer simultanément une partie de la suspension de culture contenant lesdits tissus ou cellules de la première cuve de culture vers la seconde cuve de culture ;

(e) le fait d'ajuster le débit d'alimentation (vml [l/jour]) du milieu de la première cuve de culture, la vitesse de croissance spécifique ($\mu$) desdits tissus ou cellules dans la première cuve de culture a une valeur de 0,02 à 1,0 jour$^{-1}$ et la quantité (V) du milieu de culture dans la première cuve de culture de façon que $\mu < $ vml $< V < 20\,\mu$ ; et
(f) le fait de soutirer une partie de la suspension de culture contenant lesdits tissus et cellules de la seconde cuve de culture et d'en récupérer la berbérine.

2. Procédé selon la revendication 1, dans lequel on alimente la première cuve de culture avec un milieu de culture approprié pour la production de la berbérine, ce milieu étant différent du milieu de culture alimentant la première cuve de culture.

3. Procédé selon la revendication 1 ou 2, comprenant en outre le transfert du milieu de culture ne contenant pratiquement pas de tissus ou de cellules, de la première cuve de culture vers la seconde cuve de culture, ledit milieu étant obtenu en éliminant les tissus ou cellules de la suspension de culture de la première cuve de culture.

4. Procédé selon la revendication 3, dans lequel on élimine les tissus ou cellules par filtration de la suspension de culture de la première cuve de culture.

FIG. I

FIG.2